# EUROPÄISCHE PATENTANMELDUNG

(11) **EP 1 891 989 A1**
(43) Veröffentlichungstag der Anmeldung: **27.02.2008**
(21) Anmeldenummer: 06119389.2
(22) Anmeldetag: 23.08.2006
(51) Int. Cl.: A61L 27/32

(54) **Verfahren zur Erzeugung einer degradationsresistenten Oberflächenschicht auf einem aus Titan oder aus einer Titanlegierung bestehenden Gegenstand**

(71) Anmelder: Estoppey-Reber SA, 2558 Aegerten (CH)
(72) Erfinder: Weisser, Jürgen, 99092, Erfurt (DE); Schnabelrauch, Matthias, 07749, Jena (DE); Henning, Angelika, 07751, Zöllnitz (DE); Schmidt, Jürgen, 07745, Jena (DE); Bayer, Ulrich, 07751, Jena (DE)
(74) Vertreter: BOVARD AG

(57) **Zusammenfassung**

Die Erfindung betrifft ein Verfahren zur Erzeugung einer degradationsresistenten Oberflächenschicht auf einem Gegenstand, der aus Titan oder aus einer Titanlegierung besteht, bei dem:
- eine Lösung enthaltend Calcium, Magnesium und Phosphor, wobei
• das molare Verhältnis Ca/Mg 0,05 bis 50 und
• das molare Verhältnis (Ca+Mg)/P 0,2 bis 4 betragen,
vorbereitet wird ;
- der Gegenstand in die besagte Lösung eingetaucht wird;
- der Gegenstand einer anodischer Oxidation mit einer gepulsten variablen Gleichspannung und einer konstanten Stromdichte ausgesetzt wird, bis die Gleichspannung einen Wert von mehr als 200 V erreicht.

Die Erfindung betrifft auch die Oberflächenschichten und Gegenstände, die durch solch ein Verfahren hergestellt werden.

Die Erfindung ist besonders geeignet zur Beschichtung von medizinischen Implantaten oder Dentalimplantaten.

## Beschreibung

Die Erfindung betrifft ein Verfahren zur Erzeugung einer degradationsresistenten Oberflächenschicht auf einem Gegenstand, der aus Titan oder aus einer Titanlegierung besteht, sowie die Oberflächenschichten und Gegenstände, die durch solch ein Verfahren hergestellt werden.

Die Erfindung ist besonders geeignet zur Beschichtung von medizinischen Implantaten oder Dentalimplantaten.

### Grundlage der Erfindung

Titan und Titanbasislegierungen sind gängige Implantatwerkstoffe für den Hartgewebebereich. Durch die Ausbildung einer dichten Passivierungsschicht aus Titandioxid verhalten sich Titanimplantate im Körper weitgehend inert und sind daher biokompatibel. Eine wesentliche Zielstellung der Entwicklung moderner Implantatmaterialien besteht darin, über die Biokompatibilität hinaus, ein bioaktives Verhalten der Materialien zu befördern, d. h. deren Einwachsverhalten und die Bildung eines optimalen Material-Gewebe-Verbundes positiv zu beeinflussen. Eine aussichtsreiche Methode der Generierung eines solchen bioaktiven Verhaltens wird in der Beschichtung von Implantaten mit geeigneten bioaktiven Schichtmaterialien gesehen.

### Stand der Technik

Es ist bekannt, dass die anwendungsrelevanten Eigenschaften von metallischen Implantaten wie beispielsweise die Korrosionsbeständigkeit und das Zelladhäsions- sowie Einwachsverhalten durch das Aufbringen von geeigneten Beschichtungen auf die metallischen Implantatoberflächen wesentlich verbessert werden können. Die Eigenschaftsverbesserung kann dabei sowohl durch eine geeignete chemische Zusammensetzung der Beschichtung als auch durch eine entsprechende Morphologie der Oberflächenschicht, z. B. eine die Zelladhäsion und das Einwachsen von Zellen fördernde Oberflächenporosität erreicht werden. Eine essentielle Grundvoraussetzung des Einsatzes einer solchen Beschichtung für medizinische Implantate besteht in einer ausreichenden Haftfestigkeit auf dem metallischen Substrat, so dass unter allen Umständen eine Ablösung der Schicht oder von Teilen derselben während oder nach der Implantation vermieden wird.

Dem Stand der Technik nach bekannte Oberflächenbeschichtungen bestehen z. B. aus Calciumphosphaten, da von diesen Verbindungen bekannt ist, dass sie bioaktive Eigenschaften besitzen, also die Ausbildung eines festen Verbundes zwischen Implantatoberfläche und dem umgebenden Gewebe fördern. Die Aufbringung von Calciumphosphat-Beschichtungen auf Implantatmaterialien ist auf unterschiedliche Weise möglich.

Als bekannte und in der Technik eingeführte Verfahren gelten dabei das Flammen- und Plasmaspritzen, wobei ein Nachteil dieser Verfahren in dem relativ hohen technologischen Aufwand liegt, der zur Erzielung gleichmäßiger Schichten erforderlich ist. Technologisch leichter zu realisieren sind elektrochemische Verfahren der Schichtaufbringung, insbesondere galvanische Verfahren wie die der anodischen Oxidation.

In DE 102004 022 768 wird beispielsweise eine glaskeramische Multielement-Oberflächenschicht für Implantate beschrieben, die neben Calcium, Phosphor, Sauerstoff und Fluor noch weitere Elemente wie z. B. Titan, Magnesium, Kalium oder Natrium enthält. Der Nachteil dieses Verfahrens ist darin zu sehen, dass es sich um einen zweistufigen Prozess handelt.

Ebenso wird in DD 210607 ein Verfahren zur Herstellung von beschichteten Implantaten beschrieben. Gemäss dieser Patentschrift benutzt man zur Erzeugung einer Oxidschicht eine anodische Oxidation unter Funkenentladung, wobei ein Elektrolyt verwendet wird, der keinerlei organische Bestandteile enthält, d.h., dass die Ionen nicht in organischen Komplexen gebunden sind und somit die als wirksam bezeichneten Kationen nicht durch eine anodische Kontaktierung des zu beschichtenden Werkstückes abgeschieden werden können. Es findet mehr oder weniger eine Oxidation der Implantatoberfläche statt und die im Elektrolyt vorliegenden Kationen wie Na, K, Ca und Mg wandern unter dem Einfluss des elektrischen Feldes zur Kathode der elektrolytischen Zelle.

In der internationalen Patentanmeldung WO 02/078759 wird ein galvanostatisches Verfahren beschrieben, das zur Erzeugung von bioaktiven porösen Oberflächenschichten aus amorphen und nanokristallinen Calciumphosphatphasen führt. Auf Grund der Struktur und der chemischen Zusammensetzung der abgeschiedenen Schicht aus Calcium- und Phosphat-Komponenten in einem Verhältnis, das dem der natürlichen mineralischen Knochensubstanz nahe kommt, kann die beschriebene Schicht nach der Implantation in den Knochen teilweise in Lösung gehen und zur Knochenbildung um das Implantat beitragen. Dieser an sich erwünschte Effekt ist jedoch mit zahlreichen Problemen behaftet. So ist es schwierig das Auflösungsverhalten der calciumphosphathaltigen Schicht und den Prozess der Knochenneubildung um das Implantat so zu steuern, das ein optimales Einwachsverhalten und eine optimale mechanische Stabilität des Implantats über den gesamten Zeitraum von der Implantation bis zur festen Verankerung des Implantats im Knochenlager gegeben sind. Dies ist insbesondere dadurch begründet, dass sowohl die Auflösung der Calciumphosphatphase im Körper als auch die Prozesse der Knochenneubildung von unterschiedlichen Faktoren wie z. B. dem jeweiligen Implantationsort, der Implantatgröße, dem Alter und der Konstitution des Patienten abhängen. Da eine individuelle Auslegung einer resorbierbaren bioaktiven Beschichtung mit hohen Kosten verbunden ist, erscheint es daher sinnvoll als Alternative zu teilresorbierbaren Calciumphosphatschichten gering degradierbare Beschichtungen mit gutem Einheilverhalten zu betrachten.

In DE 4303575 wird ein Verfahren zur Herstellung apatitbeschichteter Metallimplantate unter Verwendung einer plasmachemischen Reaktion mittels Wechselstrom beschrieben. Als Elektrolyt wird eine Dispersionslösung verwendet, die unter Anwendung sehr hoher Stromdichten sehr dicke Schichten erzeugt.

In US 5205921 wird ein Verfahren mit einer inerten Anode beschrieben, welches bei sehr geringen Badspannungen auf den Kathodenmaterialien Edelstahl oder Titan entsprechende Schichten erzeugt.

Die internationale Patentanmeldung WO 03/039609 beschreibt ein mehrstufiges elektrochemisches Verfahren zur Erzeugung von calciumphosphathaltigen Schichten, wobei der Elektrolyt entsprechende Ionen enthält bzw. eine Suspension darstellt.

In WO 2004/000378 werden Gradientenschichten beschrieben, die in einem Mehrstufenprozess (es kommen verschiedene Elektrolytlösungen nacheinander zum Einsatz) hergestellt werden.

Auch in WO 2005/084577 wird ein Mehrstufenprozess beschrieben. So wird das zu beschichtende Implantat in eine magnesiumhaltige Lösung getaucht und mittels UV- Bestrahlung wird dieses auf der Oberfläche verankert. Mit einer anschließenden anodischen Oxidation bei Spannungen von 60 bis 500 V wird die gewünschte Schicht gebildet.

Die Patentanmeldung CA 2073781 beschreibt in einem Zweistufenprozess die Erzeugung einer kristallinen Oxidschicht und darauf die Erzeugung einer bioaktiven Calciumphosphatschicht. Die Elektrolytlösungen liegen im pH-Wertbereich von 3 bis 8. Dabei handelt es sich um einen galvanischen Prozess bei dem zuerst in einem 30 min andauernden Intervall das Substrat zur Bildung einer Oxidschicht des Grundmaterials anodisch kontaktiert wird, in einem darauf folgenden kathodischen Teilprozess das Substrat im identischen Elektrolyt durch Potentialumkehr kathodisch geschaltet und dadurch die Bildung einer calciumphosphathaltigen Deckschicht ermöglicht wird. Eine Wiederholung dieser Prozedur führt zu einer Verdichtung dieser Schichtabfolge und zur Möglichkeit der Einstellung gewünschter Schichtdicken.

### Offenbarung der Erfindung

Der vorliegenden Erfindung lag die Aufgabe zugrunde, eine degradationsresistente und haftfeste Oberflächenbeschichtung für Titan und Titanbasislegierungen bereitzustellen, mit deren Hilfe die Biokompatibilität und das Einwachsverhalten von medizinischen und Dentalimplantaten verbessert wird.

Die Aufgabe wird erfindungsgemäß dadurch gelöst, dass auf Implantate aus Titan oder Titanbasislegierungen eine poröse Beschichtung unter Anwendung der plasmachemischen Oxidation aufgebracht wird, in der variable Anteile von Calcium- und Magnesiumphosphat-phasen nebeneinander vorliegen.

Dies erfolgt mit dem erfindungsgemässen Verfahren, bei dem:
- eine Lösung enthaltend Calcium, Magnesium und Phosphor, wobei
   • das molare Verhältnis Ca/Mg 0,05 bis 50 und
   • das molare Verhältnis (Ca + Mg)/P 0,2 bis 4 betragen;
   vorbereitet wird ;
- der Gegenstand in die besagte Lösung eingetaucht wird ;
- der Gegenstand einer anodischen Oxidation mit einer gepulsten variablen Gleichspannung und einer konstanten Stromdichte ausgesetzt wird, bis die Gleichspannung einen Wert von mehr als 200 V erreicht.

Diese Endspannung von mehr als 200 V dient zur Erzielung einer genügenden Schichtdicke. Nach Erreichen dieser Endspannung wird die Stromdichte vorzugsweise nicht mehr konstant gehalten. Dann beginnt der Strom zu sinken.

Vorteilhafterweise wird der Beschichtungsprozess z.B. manuell beendet, wenn die Stromdichte einen Wert von ca. 50% ihres Ausgangswertes (d. h. des Wertes, den sie hatte, als sie konstant war) erreicht.

Auf diese Weise werden Beschichtungen erhalten, in denen Calcium- und Magnesiumphosphatphasen in variablen Anteilen vorliegen. Solche Beschichtungen zeichnen sich durch eine weitgehende Stabilität gegenüber Lösevorgängen aus und bleiben damit über einen Zeitraum von mehreren Monaten intakt.

Gleichzeitig wurde gefunden, dass diese Beschichtungen hinsichtlich ihrer Biokompatibilität vergleichbare Eigenschaften besitzen wie eine herkömmliche teilresorbierbare calciumphosphathaltige Beschichtung.

### Ausführliche Beschreibung der Erfindung

### Erfindungsgemässes Verfahren

### a) Vorbereitung der Lösung

Erfindungsgemäss wird in einem ersten Schritt eine Lösung vorbereitet, die als Elektrolyt dient. In dieser Lösung sind die Elemente Calcium, Magnesium und Phosphor vorhanden.

Erfindungsgemäss werden in der Lösung die folgenden molaren Verhältnisse verwendet:
- 0,05 ≤ Ca/Mg ≤ 50
- 0,2 ≤ (Ca+Mg)/P ≤ 4

Vorzugsweise werden die folgenden molaren Verhältnisse verwendet:
- 0,1 ≤ Ca/Mg ≤ 25
- 0,5 ≤ (Ca+Mg)/P ≤ 2

Die Elemente Ca, Mg und P können aus verschiedenen Verbindungen kommen. Da die Lösung im Allgemeinen eine wässrige Lösung ist, stammen diese Elemente im Allgemeinen aus wasserlöslichen Verbindungen.

Als Beispiele für Verbindungen, die Calcium enthalten, seien Ethylendiamintetraessigsäuresalze, insbesondere Ethylendiamintetraessigsäure-Calcium-di-Natriumsalz-Dihydrat, Calciumdiacetat, Calciumhypophosphit genannt.

Als Beispiele für Verbindungen, die Magnesium enthalten, seien Ethylendiamintetraessigsäuresalze, insbesondere Ethylendiamintetraessigsäure-Magnesium-di-Natriumsalz, Magnesiumhypophosphithexahydrat genannt.

Als Beispiele für Verbindungen, die Phosphor enthalten, seien phosphorhaltige Verbindungen wie Phosphatsalze, insbesondere Ammoniumdihydrogenphosphat, Natriumhypophosphit Monohydrat, Kaliumdihydrogenphosphat genannt.

Vorteilhafterweise wird der pH-Wert der Lösung auf einem Wert über 7,5 eingestellt. Dies kann mit einer Base, vorzugsweise einer Ammoniaklösung erfolgen.

### b) Eintauchen des Gegenstandes bzw. des Implantats in die Lösung

Der Gegenstand wird dann anodisch kontaktiert, an eine Stromversorgung angeschlossen und in geeigneter Weise in die Elektrolytlösung eingetaucht.

### c) Anodische Oxidation

An den als Anode geschalteten Gegenstand wird dann eine gepulste Gleichspannung beginnend von 0 Volt angelegt.

Eine konstante Stromdichte von 0,5 bis 20 A/dm² wird ausgewählt.

Eine Endspannung von mehr als 200 V wird ausgewählt. Falls Schichtdicken von mehr als 2 µm erzielt werden sollen, muss die einzustellende Badspannung mehr als 250 V betragen.

Der Prozess der anodischen Oxidation beginnt bei 0 Volt Badspannung. Die Stromdichte wird konstant gehalten und man lässt die Badspannung im Laufe des Prozesses ansteigen.

Wenn die Badspannung die ausgewählte Endspannung erreicht hat, wird die Stromdichte vorzugsweise nicht mehr konstant gehalten und man lässt sie abfallen, vorzugsweise bis sie einen Wert von ca. 50% ihres früheren ausgewählten konstanten Wertes erreicht. Anschließend beendet man den Beschichtungsprozess.

Vorzugsweise beträgt die Frequenz der eingesetzten Gleichspannung von 10 bis 2000 Hz.

Die Pulse zeichnen sich durch eine steil ansteigende linke Flanke und einen etwas flacheren Verlauf der rechten Flanke des Beschichtungsstromes aus. Jeder Puls dauert vorzugsweise ca. eine halbe Periode.

Es kommt dann im Prozess der unter Funkenentladungen ablaufenden anodischen Oxidation zu komplexen Reaktionen zwischen Anodenmaterial und Elektrolytbestandteilen in deren Folge oxidische, hydroxidische und phosphathaltige Strukturen des Titans, Calciums und Magnesiums gebildet werden.

In einer bevorzugten Ausführungsform der Erfindung wird die Oxidation bei einer Elektrolyttemperatur von 5 bis 50°C durchgeführt. Dazu kann die Lösung vor oder nach dem Eintauchen des Gegenstandes erwärmt werden.

Die Dauer der anodischen Oxidation beträgt im Allgemeinen von 0,2 Minuten bis 60 Minuten, insbesondere über 10 Minuten.

### Erfindungsgemässe Schicht und Gegenstand

Im Ergebnis dieses über die Verfahrensbedingungen steuerbaren Prozesses werden festhaftende Schichten auf Titan und Titanbasislegierungen gebildet.

Durch die während des plasmachemischen Oxidationsprozesses entstehende Porenstruktur auf der Gegenstand- bzw. Implantatoberfläche erfolgt eine Vergrößerung der realen Oberfläche, so dass die resultierende porös strukturierte Oberflächenschicht ausgezeichnete Voraussetzungen für die Adhäsion von Zellen und die Ausbildung einer optimalen Grenzfläche zwischen Implantat und umliegendem Gewebe bietet.

Die degradationsresistente Schicht weist im Allgemeinen eine Dicke von 2 Mikrometern bis 50 Mikrometern auf. Vorzugsweise wird die anodische Oxidation fortgesetzt, bis die Dicke einen Wert grösser als 5 Mikrometer erreicht.

Die erhaltene mikroporige Oberflächenschicht weist eine signifikant offene Struktur auf. Sie besteht vorwiegend aus magnesium- und calciumphosphathaltigen röntgenamorphen Phasen.

Die erfindungsgemäß hergestellten Beschichtungen sind mit den üblichen Methoden sterilisierbar.

### Beispiele

Die Erfindung wird an Hand nachstehender Ausführungsbeispiele in nicht einschränkender Weise erläutert.

Alle hergestellten Schichten wurden über einen Zeitraum von 26 Wochen in SBF ("Simulated Body Fluid", auf Deutsch "Simulierte Körperflüssigkeit") bei 37°C ausgelagert.

Es wurden elektronenmikroskopisch nur äusserst geringfügige Änderungen der Oberflächenmorphologie, der Porendichte und Porengrösse sowie der Schichtdicke festgestellt.

Es wurden keine Veränderungen der chemischen Zusammensetzung der Schichten mittels energiedispersiver Röntgenanalyse festgestellt.

### Beispiel 1

Ein wässriger Elektrolyt, in dem 50 g/l Ethylendiamintetraessigsäure Calcium-di-Natriumsalz-Dihydrat (C₁₀H₁₂Na₂CaN₂O₈*2H₂O), 10 g/l Ammoniumdihydrogenphosphat (NH₄H₂PO₄), 30 g/l Ethylendiamintetraessigsäure Magnesium-di-Natriumsalz (C₁₀H₁₂Na₂MgN₂O₈) und 1 g/l Ethylendiamintetraessigsäure (EDTA) gelöst werden, wird mittels Ammoniaklösung auf einen pH- Wert von 8,6 eingestellt.

Ein Reintitanblech wird in einer wässrigen Lösung, mit 175 ml/l HNO₃ (65%-ig) und 175 ml/l HF (40%-ig) 2 bis 10 s gebeizt und im wässrigen Elektrolyten anodisch kontaktiert. Die plasmachemische Oxidation des Titanbleches erfolgt bei einer gepulsten Gleichspannung mit einer Frequenz von 1000 Hz und einer Stromdichte von 5 A/dm² bei einer Elektrolyttemperatur von 20 bis 40 °C. Die Pulse haben im Wesentlichen die Form eines spitzen Haifischzahnes mit steilem Anstieg und einer etwas flachen auslaufenden rechten Flanke.

Mit einer Endspannung von 280 V wird eine Schichtdicke von 5 bis 7 µm erzielt.

### Beispiel 2

Ein wässriger Elektrolyt, in dem 50 g/l Ethylendiamintetraessigsäure Calcium-di-Natriumsalz-Dihydrat, 5 g/l Ammoniumdihydrogenphosphat, 30 g/l Ethylendiamintetraessigsäure Magnesium-di-Natriumsalz und 1 g/l Ethylendiamintetraessigsäure gelöst werden, wird mittels Ammoniaklösung auf einen pH-Wert von 8,6 eingestellt.

Ein Reintitanblech wird in einer wässrigen Lösung, mit 175 ml/l HNO₃ (65%-ig) und 175 ml/l HF (40%-ig) 2 bis 10 s gebeizt und im wässrigen Elektrolyten anodisch kontaktiert. Die plasmachemische Oxidation des Titanbleches erfolgt bei einer gepulsten Spannung mit einer Frequenz von 1000 Hz und einer Stromdichte von 10 A/dm² bei einer Elektrolyttemperatur von 20 bis 40 °C. Die Pulse haben im Wesentlichen die Form eines spitzen Haifischzahnes mit steilem Anstieg und einer etwas flachen auslaufenden rechten Flanke.

Mit der Endspannung von 350 V wird eine Schichtdicke von 10 µm bis 15 µm erzielt.

### Beispiel 3

Ein wässriger Elektrolyt, in dem 50 g/l Ethylendiamintetraessigsäure Calcium-di-Natriumsalz-Dihydrat, 20 g/l Ammoniumdihydrogenphosphat, 30 g/l Ethylendiamintetraessigsäure Magnesium-di-Natriumsalz und 1 g/l Ethylendiamintetraessigsäure gelöst werden, wird mittels Ammoniaklösung auf einen pH-Wert von 8,6 eingestellt.

Ein Implantat aus der Titanlegierung TiAI6V4 wird in einer wässrigen Lösung, mit 175 ml/l HNO₃ (65%-ig) und 175 ml/l HF (40%-ig) 2 bis 10 s gebeizt und im wässrigen Elektrolyten anodisch kontaktiert. Die plasmachemische Oxidation dieses Implantates erfolgt bei einer gepulsten Spannung mit einer Frequenz von 2000 Hz und einer Stromdichte von 5 A/dm² bei einer Elektrolyttemperatur von 20 bis 40°C. Die Pulse haben im Wesentlichen die Form eines spitzen Haifischzahnes mit steilem Anstieg und einer etwas flachen auslaufenden rechten Flanke.

Mit der Endspannung von 280 V wird eine Schichtdicke von 5 bis 10 µm erzielt. Die so erzeugte Schicht wird durch die Legierungselemente der Titanlegierung dunkler gefärbt als die Schicht auf Reintitan.

## Patentansprüche

1. Verfahren zur Erzeugung einer degradationsresistenten Oberflächenschicht auf einem aus Titan oder einer Titanlegierung bestehenden Gegenstand, bei dem:
- eine Lösung enthaltend Calcium, Magnesium und Phosphor, wobei
• das molare Verhältnis Ca/Mg 0,05 bis 50 und
• das molare Verhältnis (Ca+Mg)/P 0,2 bis 4 betragen,
vorbereitet wird ;
- der Gegenstand in die besagte Lösung eingetaucht wird;
- der Gegenstand einer anodischer Oxidation mit einer gepulsten variablen Gleichspannung und einer konstanten Stromdichte ausgesetzt wird, bis die Gleichspannung einen Wert von mehr als 200 V erreicht.

2. Verfahren nach Anspruch 1, bei dem man danach die Stromdichte abfallen lässt.

3. Verfahren nach Anspruch 2, bei dem man die Stromdichte abfallen lässt, bis sie einen Wert von ca. 50% des Wertes, den sie hatte, als sie konstant war, erreicht.

4. Verfahren nach einem der Ansprüche 1 bis 3, bei dem der pH-Wert der Lösung vor dem Eintauschen des Gegenstandes auf einen Wert über 7,5 eingestellt wird.

5. Verfahren nach Anspruch 4, bei dem der pH-Wert der Lösung mit einer Ammoniaklösung eingestellt wird.

6. Verfahren einem der Ansprüche 1 bis 5, bei dem die gepulste Gleichspannung eine Frequenz von 10 bis 2000 Hz hat.

7. Verfahren nach einem der Ansprüche 1 bis 6, bei dem die anodische Oxidation bei einer Lösungstemperatur von 5 bis 50°C durchgeführt wird.

8. Verfahren nach einem der Ansprüche 1 bis 7, bei dem die anodische Oxidation mit Stromdichten von 0,1 bis 20 A/dm² durchgeführt wird.

9. Verfahren nach einem der Ansprüche 1 bis 8, bei dem die in der Lösung enthaltenen Calcium- und Magnesium-Elemente aus Ethylendiamintetraessigsäuresalzen stammen.

10. Verfahren nach einem der Ansprüche 1 bis 9, bei dem das in der Lösung enthaltene Phosphor aus einer phosphathaltigen Verbindung stammt.

11. Verfahren nach einem der Ansprüche 1 bis 10, bei dem eine Lösung enthaltend Calcium, Magnesium und Phosphor, wobei
• das molare Verhältnis Ca/Mg 0,1 bis 25 und
• das molare Verhältnis (Ca+Mg)/P 0,5 bis 2 betragen ;
vorbereitet wird.

12. Verfahren nach einem der Ansprüche 1 bis 11, bei dem der Gegenstand ein Implantat ist.

13. Oberflächenschicht auf Titan oder einer Titanlegierung, die durch das Verfahren gemäss einem der Ansprüche 1 bis 12 erzeugt worden ist.

14. Gegenstand aus Titan oder einer Titanlegierung, insbesondere Implantat, mit einer durch das Verfahren gemäss einem der Ansprüche 1 bis 12 erzeugten degradationsresistenten Oberflächenschicht.
